# EUROPEAN PATENT SPECIFICATION

(11) **EP 4 346 593 B1**
(45) Date of publication and mention of the grant of the patent: **09.12.2026**
(21) Application number: 22814792.2
(22) Date of filing: 15.03.2022
(51) Int. Cl.: A61B 5/145

(54) **ANALYTE DETECTION DEVICE**
ANALYTDETEKTIONSVORRICHTUNG
DISPOSITIF DE DÉTECTION D'ANALYTE

(30) Priority: 31.05.2021 WO PCT/CN2021/097173
(43) Date of publication of application: 10.04.2024
(73) Proprietor: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2022/080845
(87) International publication number: WO 2022/252744

(56) References cited:
- EP-A1- 3 203 396
- CN-A- 103 462 615
- CN-A- 104 749 228
- CN-A- 105 361 891
- CN-A- 109 998 560
- CN-A- 110 881 983
- CN-A- 112 006 653
- CN-A- 112 237 429
- CN-A- 113 274 004

## Description

### TECHNICAL FIELD

The invention mainly relates to the field of medical devices, in particular to an analyte detection device.

### BACKGROUND

The pancreas in a normal human body can automatically monitor the blood glucose level and automatically secrete the required amount of insulin/glucagon. In the body of a type 1 diabetes patient, the pancreas does not function properly and cannot produce enough insulin for the body. Therefore, type 1 diabetes is a metabolic disease caused by abnormal pancreatic function, and diabetes is a lifelong disease. At present, there is no cure for diabetes with medical technology. The onset and development of diabetes and its complications can only be controlled by stabilizing blood glucose.

Diabetics need to have their blood glucose measured before they inject insulin into the body. At present, most of the testing methods can continuously measure blood glucose level and transmit the data to a remote equipment in real time for the user to view. This method is called Continuous Glucose Monitoring (CGM).

CN 113 274 004 A discloses an analyte detection device comprising a transmitter with an electrical connection area and a bottom case with a sensor. The sensor includes a signal output end with pins electrically connected to electrodes at a detection end of the sensor. The pins and electrodes are arranged on the same side of a substrate of the sensor. The pins face toward an elastic conductor arranged between the signal output end and the electrical connection area of the transmitter. The electrodes face toward an auxiliary needle for piercing the detection end into the skin of a user.

Before the analyte detection device of prior art is installed, the substrate of the detection end of the sensor is bent to the plane away from the auxiliary needle, which is not conducive to the detection end penetrating into the user's skin and affecting the detection reliability of the sensor. The reason is that the electrode is arranged on one side of the substrate of the detection end, and the substrate will bend to the other side without electrode. The electrode and the pin on the signal output end are located on the same side of the substrate, and the pin is arranged towards the transmitter to facilitate electrical connection with the transmitter electrical connection area through the elastic conductor. Therefore, before the installation of analyte detection device, the substrate of the sensor with the electrode facing the auxiliary needle, the substrate is bent away from the auxiliary needle side.

Therefore, the prior art urgently needs a more reliable analyte detection device.

### BRIEF SUMMARY OF THE INVENTION

The object of the present invention is achieved with an analyte detection device according to claim 1. The pins are arranged on the signal output end of the sensor, the electrodes are arranged on the detection end, and the pins are connected with the electrodes through wires and arranged on the same side of the insulated sensor substrate. The pins are arranged towards the sensor base, and they are connected with the elastic conductor through the external circuit, and then electrically connected with the electrical connection area of the transmitter. After the orientation of the pin of the signal output end is changed, the side of the substrate without electrode faces the auxiliary needle, and the substrate will bend to the side close to the auxiliary needle. Due to the obstruction of the auxiliary needle, the substrate will no longer bend. When installing the analyte detection device, the auxiliary needle can smoothly pierce the sensor into the user's skin, so as to improve the detection reliability of the sensor.

The invention discloses an analyte detection device, which comprises: a transmitter provided with an electrical connection area; a bottom case provided with a sensor base; and a sensor comprising a signal output end and a detection end. The signal output end is provided with pins, the detection end is provided with electrodes, and the pins are connected with the electrodes through wires. The pins, the wire and the electrodes are arranged on an insulating substrate and located on the same side of the substrate. Further on, the analyte detection device comprises an elastic conductor comprising at least one conductive area and an insulating area distributed at intervals, wherein the conductive area is electrically connected with the electrical connection area. The pins face toward the sensor base, the side of the detection end without electrodes faces an auxiliary needle, and the pins and the elastic conductor are electrically connected through an external circuit.

According to one aspect of the invention, the elastic conductor is the cuboid structure.

According to one aspect of the invention, the first surface of the elastic conductor is in contact with the signal output end, the second surface of the elastic conductor is in contact with the external circuit, and the third surface of the elastic conductor is in contact with the electrical connection area.

According to one aspect of the invention, the first surface of the elastic conductor is in contact with the signal output end and the external circuit, and the third surface of the elastic conductor is in contact with the electrical connection area.

According to one aspect of the invention, the external circuit is a stereoscopic circuit laid on the sensor base.

According to one aspect of the invention, the external circuit is one of conductive gel, conductive adhesive, conductive coating, conductive tape or conductive glue applied to the sensor base.

According to one aspect of the invention, the first surface of the elastic conductor is opposite to the third surface.

According to one aspect of the invention, the conductive area and the insulating area pass through the elastic conductor in the longitudinal direction respectively.

According to one aspect of the invention, the conductive area and the insulating area surround the surface of the elastic conductor.

According to one aspect of the invention, the conductive area is the elastic conductor distributed at intervals, and the insulating area is the air area between the conductive areas.

According to one aspect of the invention, the elastic conductor is one of conductive adhesive strips, conductive foam or conductive bubble.

According to one aspect of the invention, the signal output end is bent relative to the detection end, and the signal output end is laid flat on the sensor base.

According to one aspect of the invention, the electrical connection area is the metal conductive contact.

According to one aspect of the invention, the number of metal conductive contact is consistent with the number of pins.

Compared with the prior art, the technical scheme of the invention has the following advantages:
In the analyte detection device disclosed by the invention, the pin is arranged towards the sensor base, the pin is connected with the elastic conductor through the external circuit, and then electrically connected with the electrical connection area of the transmitter. After the orientation of the pin of the signal output end is changed, the side of the substrate without electrode faces the auxiliary needle, and the substrate will bend to the side close to the auxiliary needle. Due to the obstruction of the auxiliary needle, the sensor substrate will no longer bend. When installing the analyte detection device, the auxiliary needle can smoothly pierce the sensor into the user's skin, so as to improve the detection reliability of the sensor.

Further, the elastic conductor comprises conductive areas and insulating areas distributed at intervals, which can realize the conduction between circuits and the insulation between different circuits at the same time. The structure is compact and convenient for the integrated design of analyte detection device.

Further, the elastic conductor is the cuboid structure, which is easy to be stably placed and fixed on the sensor base to avoid loosening and improve the reliability of the analyte detection device.

Further, the first surface of the elastic conductor is in contact with the signal output end of the sensor, that is, the elastic conductor is placed on the signal output end, which saves the installation space of the elastic conductor and is conducive to the integrated design of the analyte detection device.

Further, the second surface of the elastic conductor is in contact with the external circuit, and the third surface is in contact with the electrical connection area of the transmitter, which makes full use of the structural characteristics of the cuboid elastic conductor and is convenient for circuit design.

Further, the external circuit is a stereoscopic circuit laid on the sensor base. The stereoscopic circuit can be laid along the frame structure of the base without occupying additional space, which is conducive to the integrated design of analyte detection device.

Further, the signal output end is bent relative to the detection end, and the signal output end is laid flat on the sensor base, which can reduce the installation height of the sensor, thus reducing the overall thickness of the analyte detection device and increasing the user experience.

### BRIEF DESCRIPTION OF THE DRAWINGS

Fig.1 is the three-dimensional structural diagram of the bottom case according to an embodiment;
Fig.2a is the assembly diagram of sensor being mounted on the sensor base according to an embodiment;
Fig.2b is the structural diagram of the single-sided electrode sensor according to an embodiment;
Fig.3 is a three-dimensional structural diagram of the transmitter according to an embodiment;
Fig.4a-Fig.4b are structural diagrams of elastic conductor, pin and electrical connection area according to an embodiment, Fig.4a is the top view, and Fig.4b is the side view of the structure of Fig.4a;
Fig.4c is the top view structural diagram of the elastic conductor and the pin according to another embodiment;
Fig.4d-Fig.4e are the top view structural diagrams of the elastic conductor, pin and electrical connection area according to different embodiments;
Fig.5 is the structural diagram of an elastic conductor, a pin and an electrical connection area according to another embodiment;
Figs.6a- Fig.6b are the structural diagrams of the electrical connection area and the electrical connection position of the elastic conductor according to different embodiments;
Fig.7a-Fig.7b are the structural diagrams of the electrical connection of the elastic conductor with the pin and the electrical connection area respectively according to another embodiment, and Fig.7b is a cross-sectional view obtained along the section line A-A' in Fig.7a;
Fig.8a-Fig.8b are the structural diagrams of the elastic conductor electrically connected with the pin and the electrical connection area respectively according to another embodiment, and Fig.8b is the cross-sectional view obtained along the section line B-B' in Fig.8a;
Fig.9a is the three-dimensional structural diagram of the electrical connection area according to another embodiment;
Fig.9b is the three-dimensional structural diagram of the elastic conductor matched with the electrical connection area in Fig.9a and the signal output end;
Fig.10 is the structural diagram of the signal output end arranged on the top of an elastic conductor according to another embodiment;
Fig.11 is the structural diagram in which the signal output end of different parts is arranged at different positions of the elastic conductor according to another embodiment.
Fig.12a is the schematic diagram of the bending of the detection end when the sensor is installed on the base according to the embodiment;
Fig.12b is the schematic diagram of the detection end when the sensor is upside down on the base according to the embodiment;
Fig.13 is the assembly diagram of the sensor upside down on the base according to the embodiment;
Fig.14 is the structural diagram of the stereoscopic electrode sensor according to an embodiment;
Fig.15 is the schematic diagram of the stereoscopic electrode sensor installed on the base according to an embodiment.

### DETAILED DESCRIPTION

As mentioned above, before the analyte detection device of prior art is installed, the substrate of the detection end is bent to the plane away from the auxiliary needle, which is not easy to penetrate the user's skin during installation, affecting the detection reliability of the sensor.

In order to solve this problem, the invention provides an analyte detection device, the pin is arranged towards the sensor base, the pin is connected with the elastic conductor through the external circuit, and then electrically connected with the electrical connection area of the transmitter. After the orientation of the pin of the signal output end is changed, the side of the substrate without electrode faces the auxiliary needle, and the substrate will bend to the side close to the auxiliary needle. Due to the obstruction of the auxiliary needle, the sensor substrate will no longer bend. When installing the analyte detection device, the auxiliary needle can smoothly pierce the sensor into the user's skin, so as to improve the detection reliability of the sensor.

Various exemplary embodiments of the invention will now be described in detail with reference to the attached drawings. It is understood that, unless otherwise specified, the relative arrangement of parts and steps, numerical expressions and values described in these embodiments shall not be construed as limitations on the scope of the invention.

In addition, it should be understood that the dimensions of the various components shown in the attached drawings are not necessarily drawn to actual proportions for ease of description, e. g. the thickness, width, length or distance of some elements may be enlarged relative to other structures.

The following descriptions of exemplary embodiments are illustrative only and do not in any sense limit the invention, its application or use. Techniques, methods and devices known to ordinary technicians in the relevant field may not be discussed in detail here, but to the extent applicable, they shall be considered as part of this manual.

It should be noted that similar labels and letters indicate similar items in the appending drawings below, so that once an item is defined or described in one of the appending drawings, there is no need to discuss it further in the subsequent appending drawings.

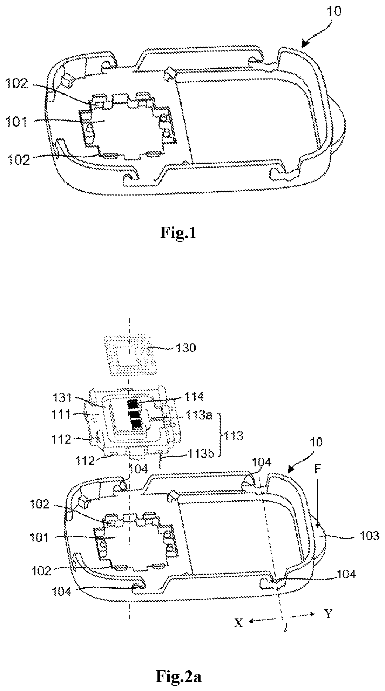

Fig.1 is the three-dimensional structural diagram of the bottom case 10 according to the embodiment of the invention. Fig.2a is the assembly diagram of the sensor 113 being installed on the base 111 according to the embodiment of the invention. A detailed description will be given with reference to Fig.1 and Fig.2A.

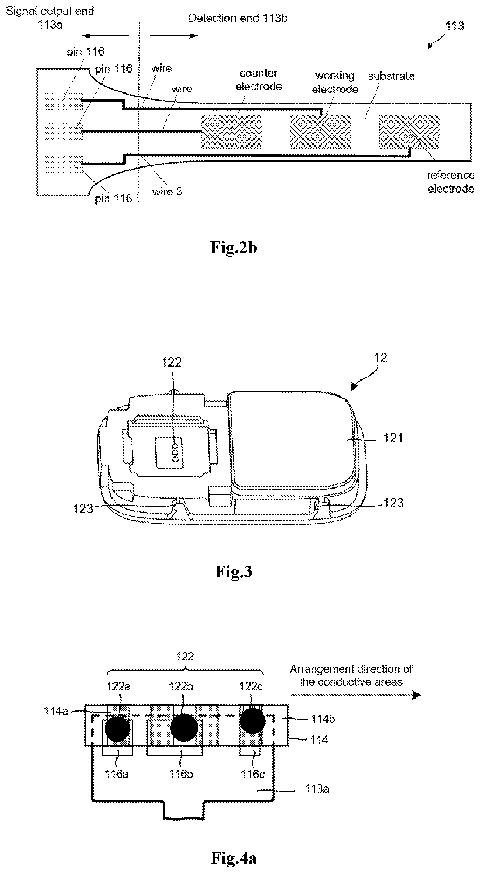

The bottom case 10 is used to assemble the sensor 113 and the transmitter 12. In the embodiment of the invention, the bottom surface of the bottom case 10 is provided with an assembly hole 101 for assisting in the installation of the sensor 113, and a first clamping structure 102 is arranged around the assembly hole 101 to assist in the installation of the sensor 113 on the bottom case 10. In combination with Fig.2a and Fig.3, the side wall of the bottom case 10 is also provided with a second clamping part 104 for fixing the transmitter 12. Correspondingly, the transmitter 12 is provided with a first clamping part 123 that can be engaged with the second clamping part 104.

In the embodiment of the invention, the number of the second clamping part 104 is two, and the two second clamping parts 104 are correspondingly arranged on the side wall of the bottom case 10.

In other embodiments of the invention, the number of the second clamping part 104 is four, and the four second clamping parts 104 correspond to the side wall opposite to the bottom shell 10, two on each side.

In other embodiments of the invention, the number of the second clamping parts 104 is six, and the six second clamping parts 104 are arranged corresponding to the side wall of the bottom case 10, two on each side.

In the embodiment of the invention, bending the force application part can invalidate the bottom case. The failure mode of the bottom case comprises one or more combinations of bottom plate fracture, bottom case fracture, second clamping part fracture and bottom case deformation of the bottom case 10. In short, after the bottom case fails, the first clamping part 123 is disengaged from the second clamping part 104, and the transmitter 12 can be disengaged from the bottom case 10. Due to the electrical connection parts, the electrical connection area 122 of the transmitter 12 is not located in the force application part, and the user will not damage the electrical connection parts when removing the transmitter 12. After replacing the bottom case 10, install the transmitter 12 on the new bottom case, which will not affect the electrical connection between the electrical connection area 122 and the pin 116 of the new bottom case, so as to ensure the stability of the electrical connection.

In the embodiment of the invention, the fixed part and the force application part are relative concepts. According to the structural design of the bottom case 10 and the transmitter 12, the positions of the fixed part and the force application part can be selected differently.

In other embodiments of the invention, the connecting line *l* of the two second clamping parts 104 divides the bottom case 10 into X side And Y side. The Y side is provided with a force application part, and the X side is provided with a fixed part.

Therefore, in the embodiment of the invention, the process of separating the bottom case 10 and the transmitter 12 is as follows: fix the fixed part on the X side with a finger, apply a force F to the force application part on the Y side with another finger in one direction, so as to invalidate the second clamping part 104, and then separate the second clamping part 104 and the first clamping part 123, so as to separate the transmitter 12 from the bottom case 10.

In the embodiment of the invention, the force application part is the protrusion 103 outward from the side of the bottom case. The shape, size and quantity of the protrusion 103 are not limited. Preferably, the protrusion 103 is a semi-circular arc, which is convenient for users to press with their fingers, saves space and ensures a small and compact bottom case structure.

In other embodiments of the invention, the bottom case 10 can also be other shapes, as long as the conditions for installing the transmitter 12 and the sensor 113 on the bottom case 10 can be met, and there is no specific limitation here.

There are many ways in which the sensor 113 can be assembled on the bottom case 10, and there are no specific restrictions here. Specifically, in the embodiment of the invention, the bottom case 10 comprises a sensor base 111. The sensor 113 is mounted on the chassis 10 through the sensor base 111. A second clamping structure 112 is arranged around the sensor base 111. The second clamping structure 112 will engage with the first clamping structure 102 to install the sensor base 111 in the assembly hole 101, and then assemble the sensor 113 on the bottom case 10.

In another embodiment of the invention, after the sensor 113 is installed on the bottom case 10, the auxiliary installation structure of the sensor 113 is removed, and the sensor 113 is not carried by the sensor base 111 or other components, but is separately installed on the bottom case 10.

In other embodiments of the invention, the sensor 113 can also be assembled on the bottom case 10 in other assembly methods, and there is no specific limitation here.

It should be noted that in the embodiment of the invention, the sensor base 111 is also provided with a sealing ring 130 and a groove 131 for placing the sealing ring 130.

Fig.2b is the structural diagram of single-sided electrode sensor. Referring to Fig.2a and Fig.2b, the sensor 113 comprises a signal output end 113a and a detection end 113b. The signal output end 113a needs to be electrically connected with the electrical connection area 122 of the transmitter 12 to transmit the detection signal to the transmitter 12. The detection end 113b is used to penetrate the subcutaneous tissue of the human body to detect the parameter information of body fluid analyte.

The signal output end 113a is provided with mutually insulated pins 116. Conventionally, the sensor 113 is also provided with electrodes and/or electrode wires for detecting analyte parameter information. The detection signal of the electrode needs to be derived through pin 116.

It should be noted that the embodiment of the invention does not limit the setting mode of pin 116 on signal output end 113a. For example, the pin 116 may be arranged on the surface of the signal output end 113a or embedded in the signal output end 113a.

Generally, the sensor 113 is provided with at least two detection electrodes, that is, it comprises at least a working electrode and a counter electrode. Therefore, in the implementation of the invention, at least two pins 116 are arranged on the surface of the signal output end 113a to be electrically connected with different electrodes. Specifically, in the embodiment of the invention, the sensor 113 is a three-electrode system, that is, a working electrode, a counter electrode and a reference electrode. Therefore, the number of pins 116 is three.

As shown in Fig.2a, in the embodiment of the invention, the signal output end 113a is bent or bent towards the bottom surface of the bottom case 10. The signal output end 113a fits with the surface of the sensor base 111 or is embedded in the sensor base 111. This design reduces the height of the sensor 113 protruding from the bottom case 10 and reduces the thickness of the detection device.

In other embodiments of the invention, the sensor 113 can also be other shapes or forms (such as non-bending), which is not specifically limited here.

Fig.3 is a three-dimensional structural diagram of the transmitter 12 according to the embodiment of the invention.

The transmitter 12 is provided with mutually insulated electrical connection areas 122. The electrical connection area 122 is used for electrical connection with the pin 116 to receive an electrical signal from the sensor 113. Therefore, the electrical connection area 122 corresponds to the pin 116.

Here, correspondence means that the number of the two is equal and their positions are basically corresponding. Obviously, in the embodiment of the invention, the number of electrical connection areas 122 is three to adapt to the three-electrode system of the sensor 113.

In the embodiment of the invention, the electrical connection area 122 is electrically connected out of the exposed shell 121. Specifically, in the embodiment of the invention, the electrical connection area 122 is the metal conductive contact. Smaller metal conductive contacts make the internal structure of the detection device more compact, and the volume of the detection device will be further reduced.

In the embodiment of the invention, the battery (not shown in the figure) is arranged in the transmitter shell 121. In other embodiments, the battery can also be arranged in the bottom case 10 to provide electric energy to the transmitter 12.

It should be noted that the embodiment of the invention does not limit the shape and position of the electrical connection area 122. For example, in one embodiment of the invention, the electrical connection area 122 does not protrude from the surface of the transmitter shell 121, but is flush with the surface of the transmitter shell 121. In another embodiment of the invention, the electrical connection area 122 is located inside the transmitter shell 121, which will be described in detail below. As in another embodiment of the invention, the section of the electrical connection area is rectangular or circular. In another embodiment of the invention, the conductive part of the electrical connection area is arranged on the surface of the connector, or the electrical connection area 122 itself is the connector. The connector can be inserted into the same elastic conductor, which will be described in detail below.

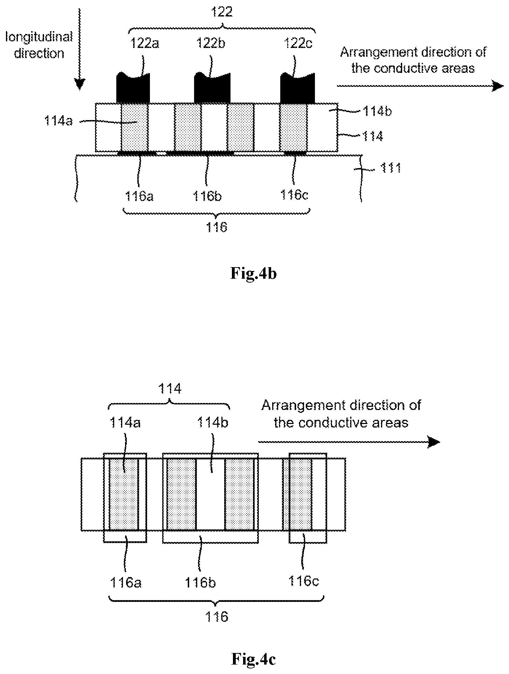

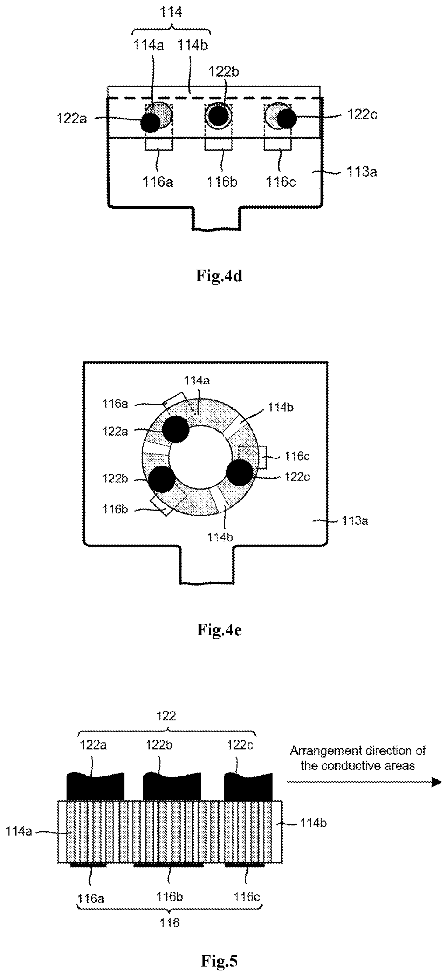

Fig.4a is the top view structural diagram of an elastic conductor, pin and electrical connection area according to the embodiment of the invention. Fig.4b is the side view of the elastic conductor in Fig.4a. Fig.4c is the top view structural diagram of an elastic conductor and a pin according to another embodiment of the invention. Fig.4d-Fig.4e are top structural diagrams of elastic conductors, pins and electrical connection areas in different embodiments of the invention.

First of all, it should be noted that in Fig.4a, the thin dotted line represents the outline of the part where the pin is covered by the elastic conductor, and the thick dotted line represents the outline of the part where the signal output end is covered by the elastic conductor. The thin dotted line and thick dotted line in the subsequent drawings have the same meaning as here, and will not be repeated below.

The detection device of the embodiment of the invention comprises an elastic conductor 114. The elastic conductor 114 is in contact with the signal output end 113a. Setting only one elastic conductor 114 reduces the number of internal structures of the detection device. In addition, the elastic material deforms after being extruded, and then plays a locking role. Therefore, the elastic conductor 114 can be connected closer to each other as a conductive structure or as an auxiliary structure at the electrical connection position, so as to improve the reliability of the electrical connection.

In one embodiment of the invention, the signal output end 113a is arranged at the bottom (first surface) of the elastic conductor 114, and the pin 116 is indirectly electrically connected with the corresponding electrical connection area 122. Here, the bottom of the elastic conductor 114 refers to the part of the elastic conductor 114 close to the skin.

At this time, the elastic conductor 114 comprises at least two conductive areas 114a and at least one insulating area 114b. The conductive area 114a and the insulating area 114b function as conductive conduction and electrical insulation, respectively. The conductive area 114a and the insulating area 114b cannot be separated from each other, that is, the conductive area 114a and the insulating area 114b belong to an integral part of the elastic conductor 114 respectively.

An insulating area 114b is arranged between adjacent conductive areas 114a. Different pins 116 or different electrical connection areas 122 are electrically connected with different conductive areas 114a respectively, so that any two pins 116 or any two electrical connection areas 122 are electrically insulated from each other.

Inside the elastic conductor 114, the conductive area 114a and the insulating area 114b pass through the elastic conductor 114 in the longitudinal direction, as shown in Fig.4b. Here, the longitudinal direction refers to the direction from the pin 116 to the corresponding electrical connection area 122, or the direction of the current between the pin 116 and the electrical connection area 122. When the pin 116 is electrically connected with the electrical connection area 122, such a design ensures that the elastic conductor 114 can only conduct longitudinal conduction, not transverse conduction. While the elastic conductor 114 electrically connects the pin 116 with the corresponding electrical connection area 122, it also electrically insulates different pins 116 or different electrical connection areas 122. An elastic conductor 114 plays the role of conduction and electrical insulation at the same time. The complexity of the internal structure of the detection device is reduced, the internal structure is more compact, and the electrical connection reliability of the detection device is improved.

It should be noted that in other embodiments of the invention, the conductive area 114a or the insulating area 114b can also have a certain inclination, or be arranged inside the elastic conductor 114 in other directions or ways. There is no specific limitation here, as long as the above conditions of conductive conduction and electrical insulation can be met.

Please refer to Fig.2a, Fig.4a and Fig.4b. Specifically, in the embodiment of the invention, the elastic conductor 114 is a cuboid structure. The conductive area 114a and the insulating area 114b are arranged at intervals and pass through the elastic conductor 114 respectively. In another embodiment of the invention, different conductive areas 114a are arranged in the same insulating area 114b, that is, surrounded by the same insulating area 114b, as shown in Fig.4d. In another embodiment of the invention, the top view of the elastic conductor 114 may be circular, as shown in Fig.4e. In another embodiment of the invention, the top view of the elastic conductor 114 may also be circular.

In other embodiments of the invention, the elastic conductor 114 can also have other shapes, which are not specifically limited here, as long as the conditions for realizing the above functions of the elastic conductor 114 can be met.

Please continue to refer to Fig.4a and Fig.4b. When the elastic conductor 114 is electrically connected with the pin 116 and the electrical connection area 122 respectively, there is an insulating area 114b between any two pins 116 connected with the elastic conductor 114. Specifically, in the embodiment of the invention, the insulating area 114b separated between any two pins 116 comprises a part of an insulating area 114b (between 116a and 116b of Fig.4a and Fig.4b), or an insulating area 114b, or more than one insulating area 114b (between 116c and 116b of Fig.4a and Fig.4b). Similarly, the insulating area 114b separated between any two electrical connection regions 122 connected to the elastic conductor 114 comprises a part of one insulating area 114b, one insulating area 114b, or more than one insulating area 114b. However, it is obvious that the pin and the corresponding electrical connection area (such as between 116a and 122a, between 116b and 122b, or between 116c and 122c) share a common part of the conductive area 114a to realize the conductivity of the two. The conductive area of the common part comprises a part of a conductive area 114a (as between 116c and 122c in Fig.4a and Fig.4b), or a conductive area 114a, or more than one conductive area 114a.

In combination with Fig.4a and Fig.4b, it is easy for those skilled in the art to understand that a part of the above insulating area or conductive area, an insulating area or conductive area, and more than one insulating area or conductive area are only the span range of the pin or electrical connection area in one-dimensional direction (such as the arrangement direction of the conductive area).

In other embodiments of the invention, a part of an insulating area or conductive area, an insulating area or conductive area, and more than one insulating area or conductive area can also represent the coverage of the pin or electrical connection area to the insulating area or conductive area in the two-dimensional direction (in area), as shown in Fig.4c. Taking the pin as an example, the dotted line in Fig.4c represents the partial outline of the pin. Obviously, the pin 116 can cover a part of an insulating area or conductive area, or an insulating area or conductive area, or more than one insulating area or conductive area.

Obviously, when the number of conductive areas or insulating areas between the above structures is large or the range is wide, the reliability of electrical connection or electrical insulation between structures will be significantly improved.

In the embodiment of the invention, the material of the elastic conductor 114 comprises elastic plastic, elastic rubber, etc. Using the elastic conductor 114 can obtain better electrical contact and buffer at the same time. When the material of the elastic conductor 114 is elastic rubber, the elastic conductor 114 is an elastic conductor. An elastic conductor not only plays the role of conduction and insulation, but also plays the role of buffer.

Obviously, when the sensor 113 is a two-electrode system, the number of pins and electrical connection areas is 2. At this time, the elastic conductor 114 only comprises two conductive area 114a and an insulating area 114b arranged between the two conductive area 114a. That is, two pairs of different pins and electrical connection areas are electrically connected through different conductive areas 114a to realize conductivity. At the same time, two pins or two electrical connection areas are separated by insulation areas to achieve electrical insulation.

The sensor of other embodiments of the invention may also comprise more electrodes. Therefore, the elastic conductor 114 comprises more conductive areas and insulating areas arranged at intervals, and the mode of electrical connection will be more flexible, as shown in Fig.5.

It should be noted that in other embodiments of the invention, the sensor comprises at least three-electrodes, that is, the signal output end 113a is provided with at least three pins, of which at least two pins are electrically connected with the corresponding electrical connection area through different conductive areas 114a, and the connection method and principle are consistent with the above. For other pins and electrical connection areas not connected with the elastic conductor 114, the embodiment of the invention does not limit the connection mode or connection principle. For example, in one embodiment of the invention, the sensor is a three-electrode system, in which only the working electrode and the counter electrode are electrically connected with the electrical connection area through the above elastic conductor through the corresponding pins, while the reference electrode is electrically connected with the transmitter through other ways.

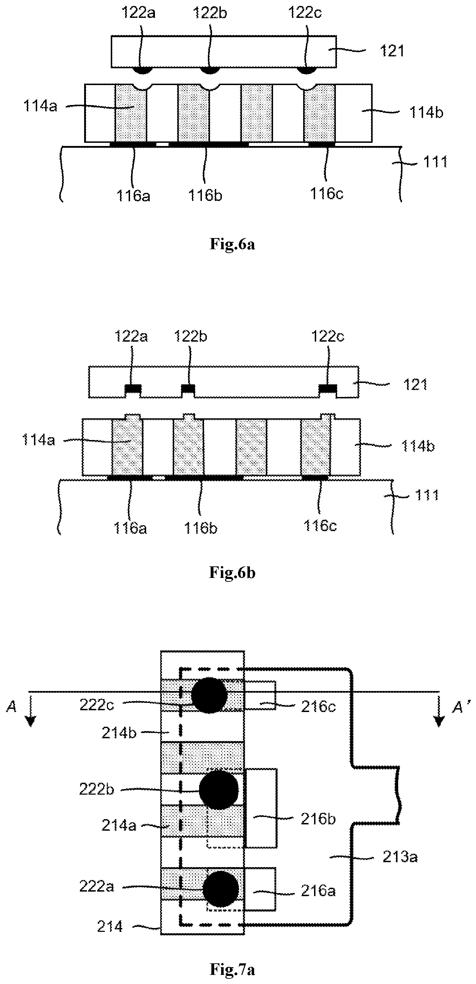

Fig.6a-Fig.6b are structural diagrams of the electrical connection positions of the electrical connection area 122 and the elastic conductor 114 in different embodiments of the invention.

For convenience of annotation and description, the electrical connection area 122 and the elastic conductor 114 in Fig.6a and Fig.6b will be shown separately.

As shown in Fig.6a, in the embodiment of the invention, the electrical connection area 122 is a convex spherical crown type metal conductive contact. Correspondingly, the elastic conductor 114 is provided with a concave part (not shown) at the position connected with the protruding metal conductive contact to make the connection closer. At the same time, the connection between the convex part and the concave part also plays a role in fixing the position of the elastic conductor 114, that is, no matter what external force the detection device receives, the position of the elastic conductor 114 is always fixed without displacement, so as to ensure that the elastic conductor 114 performs normal conduction and insulation work.

It should be noted that the elastic conductor 114 may not design a concave part. When pressed by the protruding metal conductive contact, the concave part matching with the metal conductive contact will automatically appear on the elastic conductor 114 to ensure the function of electrical connection or electrical insulation.

As shown in Fig.6b, in another embodiment of the invention, the electrical connection area 122 is arranged inside the transmitter 12. At this time, the elastic conductor 114 is correspondingly provided with a convex part (not shown), which can enter the interior of the transmitter 12 and be electrically connected with the corresponding electrical connection area 122.

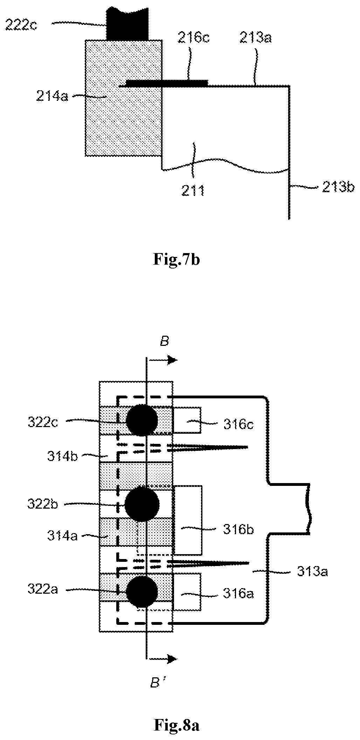

Figs.7a-7b are structural diagrams of the elastic conductor 214 electrically connected with the pin and the electrical connection area respectively in another embodiment of the invention. Fig.7a is a top view. Fig.7b is a cross-sectional view obtained along section line A-A' in Fig.7a.

The three electrical connection areas 222a, 222b and 222c of the embodiment of the invention are indirectly electrically connected with the three pins 216a, 216b and 216c respectively. The arrangement of the conductive area 214a and the insulating area 214b in the elastic conductor 214 is described above.

Specifically, please refer to Fig.7b. In the embodiment of the invention, the signal output end 213a is embedded in the elastic conductor 214. Therefore, the three pins 216a, 216b and 216c are embedded in the elastic conductor 214. In order to fix the position of the sensor, the signal output end 213a and the detection end 23b are carried by the sensor base 211.

In the embodiment of the invention, the elastic conductor 214 is electrically connected with the pin and the electrical connection area respectively, and the principle and method are consistent with the above.

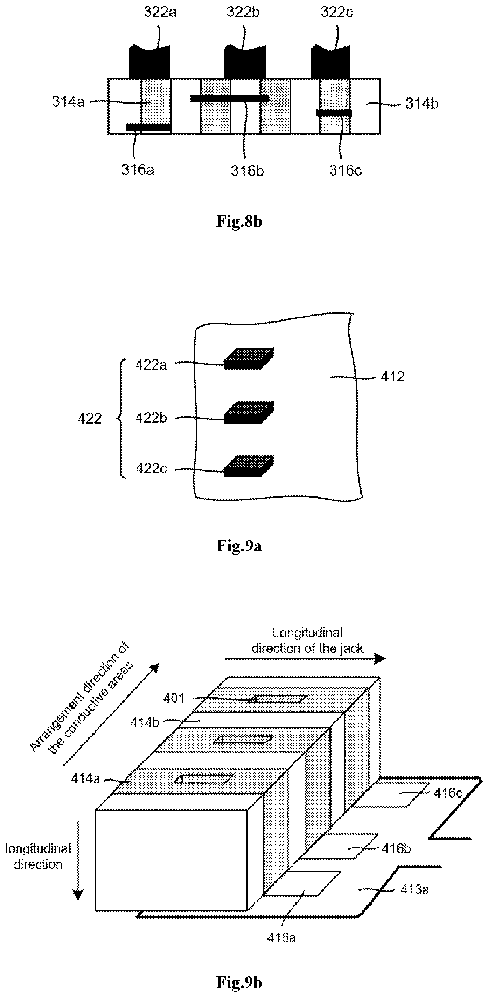

Figs.8a-8b are structural diagrams of the elastic conductor electrically connected with the pin and the electrical connection area respectively in another embodiment of the invention. Fig.8a is a top view. Fig.8b is a cross-sectional view obtained along section line B-B' in Fig.8a.

In the embodiment of the invention, different pins are arranged on different parts of the signal output end 313a, and different parts of the signal output end 313a are independent of each other and do not interfere with each other. Specifically, the three pins are embedded in the conductive area 314a and/or the insulating area 314b of the elastic conductor. As shown in Fig.8b, in the embodiment of the invention, the heights of the embedded positions of the three pins in the elastic conductor are not exactly the same.

In the actual manufacturing process, the thickness of each pin will be different. When the transmitter is connected with the sensor, the mutually independent and non-interference pins can weaken or eliminate the influence of poor contact caused by the above thickness difference, and improve the reliability of the electrical connection of the three.

Obviously, in other embodiments of the invention, only two of the three pins can be embedded in the elastic conductor, the other pin is arranged at the bottom of the elastic conductor, or the embedded height of the three pins in the elastic conductor is equal, which is not specifically limited here.

Fig.9a is the three-dimensional structural diagram of the electrical connection area 422 according to another embodiment of the invention. Fig.9b is the three-dimensional structural diagram of the elastic conductor matched with the electrical connection area 422 in Fig.9a and the signal output end 413a.

The three electrical connection areas 422a, 422b and 422c are connectors and protrude from the transmitter shell 412. The types of connectors are described above. Three jacks 401 are arranged in the elastic conductor to cooperate with the three electrical connection areas. The three electrical connection areas can be inserted into the corresponding jack 401 respectively.

In the embodiment of the invention, the length direction of the jack 401 is perpendicular to the arrangement direction of the conductive area 414a or the insulating area 414b. In other embodiments of the invention, the two directions can be designed arbitrarily according to the requirements. For example, in one embodiment of the invention, the length direction of the jack is parallel to the arrangement direction of the conductive area. Please refer to the above for the principle and method of electrical connection.

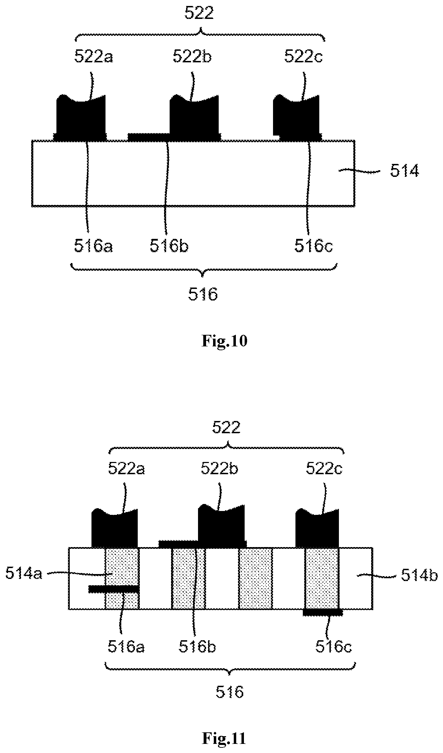

Fig.10 is the structural diagram of a signal output end arranged on the top of the elastic conductor 514 according to another embodiment of the invention.

In another embodiment of the invention, the signal output end is arranged on the top of the elastic conductor 514, that is, the signal output end is arranged between the elastic conductor 514 and the electrical connection area 522. At this time, the electrical connection area 522 is directly electrically connected with the corresponding pin 516. Therefore, the elastic conductor 514 can be an ordinary elastic conductor or the above elastic conductor provided with a conductive area. Preferably, the electrical connection area 522 is a protruding metal conductive contact. Since the elastic conductor 514 is carried below the pin 516, the reliability of the electrical connection between the electrical connection area 522 and the pin 516 is high. Similarly, the shape selection of the elastic conductor 514 can be consistent with the above and will not be repeated here.

As mentioned earlier, different parts of the signal output end can be independent of each other and do not interfere with each other. Preferably, in another embodiment of the invention, three pins 516 are respectively arranged in different parts of the signal output end. Therefore, three different parts of the signal output end are respectively arranged at different positions of the elastic conductor. For example, pin 516b is arranged at the top of the elastic conductor, pin 516a is embedded in the elastic conductor 514, and pin 516c is arranged at the bottom of the elastic conductor, as shown in Fig.11. When there are more independent pins 516, the positions of different pin settings can be arbitrarily selected as needed.

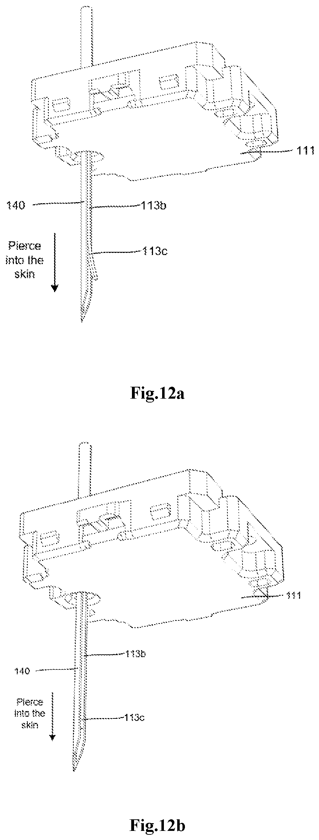

Fig.12a is the schematic diagram of the bending of the detection end when the sensor is installed on the base 111, and Fig.12b is the schematic diagram of the detection end when the sensor according to the invention is installed upside down on the base 111.

When the sensor 113 is mounted on the base 111, the pin 116 on the signal output end 113a faces the elastic conductor 114, so the electrode on the detection end 113b faces the auxiliary needle 140. Before installing the analyte detection device, the substrate 113c of the detection end 113b will bend to the side without electrode to form a curved shape as shown in Fig.12a, and the end of the detection end 113b is far away from the auxiliary needle 140. When installing the analyte detection device, the curved detection end cannot closely fit the auxiliary needle 140, and the auxiliary needle 140 cannot smoothly pierce the detection end 113b into the user's skin, affecting the detection reliability of the sensor 113.

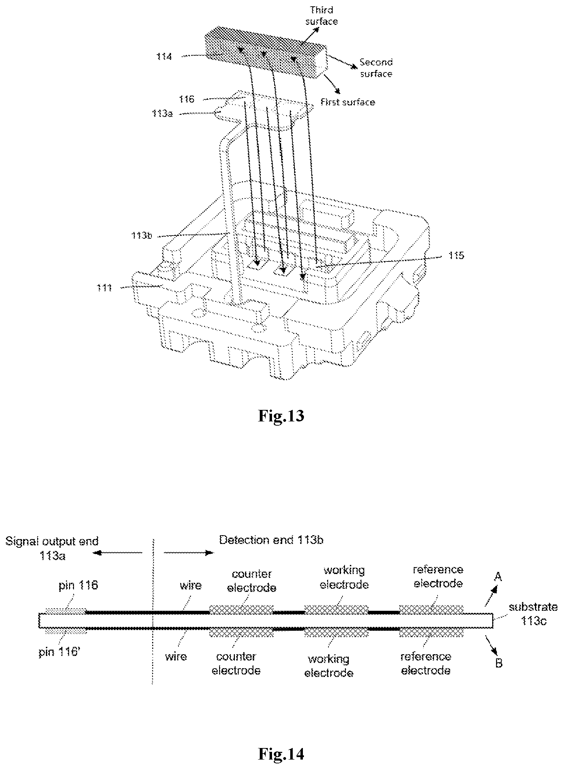

Fig.13 is an assembly diagram of the sensor upside down on the base 111 according to the invention.

In order to avoid bending of the detection end 113b before installing the analyte detection device, in the embodiment of the invention, the sensor 113 is upside down on the base 111, that is, the pin 116 faces the base 111, and the side of the detection end 113b without electrode faces the auxiliary needle 140. At this time, the substrate 113c of the detection end 113b will bend to the side of the auxiliary needle 140, but due to the obstruction of the auxiliary needle 140, The substrate 113c of the detection end 113b is no longer bent to achieve the effect shown in Fig.12b.

In the embodiment of the invention, after the sensor 113 is upside down, the pin 116 is no longer in direct contact with the first surface of the elastic conductor 114. Therefore, the external circuit 115 is also required to realize the electrical connection between the pin 116 and the elastic conductor 114.

In one embodiment of the invention, the external circuit 115 is a stereoscopic circuit laid on the sensor base 111. One end of the stereoscopic circuit is in contact with the pin 116 and the other end is in contact with the second surface of the elastic conductor 114. Both ends of the stereoscopic circuit are bent to adapt to the stereoscopic structure of the first and second surface of the elastic conductor 114, that is, the stereoscopic circuit realizes the electrical connection between the pin 116 and the second surface of the elastic conductor 114. At the same time, the third surface of the elastic conductor 114 is electrically connected with the electrical connection area 122 of the transmitter, and the third surface is the surface opposite to the first surface. Since the conductive area surrounds the elastic conductor 114, when the sensor 113 is upside down, the electrical connection between the pin 116 and the electrical connection area 122 is indirectly realized through the stereoscopic circuit and the first, second and third surfaces of the elastic conductor 114.

In the embodiment of the invention, the stereoscopic circuit can be made by LDS process, so as to realize the conductive line attached to the base frame on the sensor base 111 made of plastic.

In another embodiment of the invention, the external circuit 115 is the conductive gel coated on the sensor base 111. The conductive gel is suitable for smearing on the flat surface and can not make use of the second surface of the elastic conductor 114. Therefore, in the embodiment of the invention, the elastic conductor 114 can be widened, or the signal output end 113a of the sensor 113 can be narrowed, so that the first surface of the elastic conductor 114 can touch one end of the conductive gel, while the other end of the conductive gel is connected with pin 116, while the third surface of the elastic conductor 114 contacts with the electrical connection area 122 of the transmitter. Similarly, the electrical connection between pin 116 and electrical connection area 122 can be realized indirectly.

In the embodiment of the invention, the number of metal conductive contacts in the electrical connection area 122 is three, which is consistent with the number of pins 116 of the sensor 113, and each pin 116 corresponds to a metal conductive contact.

Fig.14 is the structural diagram of the stereoscopic electrode sensor.

Refer to Fig.14. In the embodiment of the invention, the stereoscopic electrode sensor comprises at least two groups of electrodes, which are respectively arranged on two planes (plane A and plane B) of the substrate 113c. At least two groups of electrodes can realize the functions of electrode relay and redundant detection, so as to prolong the service life of the sensor and improve the detection reliability of the sensor.

In some embodiments of the invention, each group of electrodes can be a two-electrode system, which is composed of working electrode and counter electrode. The working electrode and counter electrode can be arranged on the same side of the substrate or on both sides of the substrate, and are respectively connected with pin 116 (or pin 116') on the same side of the electrode through wires. In other embodiments of the invention, each group of electrodes can be a three-electrode system, which is composed of working electrode, counter electrode and reference electrode. The working electrode, counter electrode and reference electrode can be arranged on the same side of the substrate or on both sides of the substrate, and are respectively connected with the pin 116 (or pin 116') on the same side of the electrode through the wire.

In the embodiment of the invention, whether it is a two-electrode system or a three-electrode system, the pins 116 are distributed on both sides of the sensor substrate 113c. When the elastic conductor 114 contacts the sensor signal output end 113a, it can only contact the pins 116' on plane A, but not the pins 116 on plane B. therefore, the external circuit 115 is also required to realize the electrical connection between the pins 116 on plane B and the elastic conductor 114.

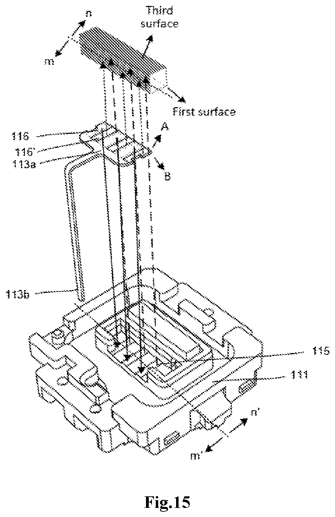

Fig.15 is a schematic diagram of a stereoscopic electrode sensor mounted on the base 111.

Refer to Fig.13 and Fig.15 in combination. In some embodiments of the invention, the external circuit 115 is a stereoscopic circuit laid on the sensor base 111. One end of the stereoscopic circuit is in contact with the pin 116 on the plane B of the sensor substrate, the other end is in contact with the second surface of the elastic conductor 114, and the pin 116' on the plane A of the sensor substrate is in contact with the first surface of the elastic conductor 114, The third surface of the elastic conductor 114 is in contact with the electrical connection area of the transmitter, and the third surface is the surface opposite to the first surface. The two ends of the stereoscopic circuit are bent to adapt to the stereoscopic structure of the first and second surface of the elastic conductor 114. The conductive area and insulating area of the elastic conductor 114 pass through the elastic conductor in the longitudinal direction, or surround the surrounding surface of the elastic conductor, or the conductive area is an elastic conductor distributed at intervals, and the insulating area is a spatial area in the middle of the conductive area, such as air or vacuum. That is, the stereoscopic circuit indirectly realizes the electrical connection between the pin 116 (116') on both sides of the sensor and the electrical connection area 122 of the transmitter through the elastic conductor 114.

Referring to Fig.15, in other embodiments of the invention, the external circuit 115 is one of the conductive adhesives coated on the sensor base 111, conductive paste, conductive coating, conductive tape or conductive glue. In the preferred embodiment of the invention, the external circuit 115 is a conductive gel coated on the sensor base 111. As mentioned above, the elastic conductor 114 is widened, or the signal output end 113a of the sensor 113 is narrowed, so that the n side of the first surface of the elastic conductor 114 contacts the n' side of the conductive gel, while the m' side of the conductive gel contacts the pin 116 of plane B of the sensor, while the m side of the first surface of the elastic conductor 114 contacts the pin 116' on the plane A of the sensor, and the third surface contacts the electrical connection area 122. Thus, the connection between the sensor double-sided pin 116 (116') and the elastic conductor 114 is achieved by conducting the gel, and then electrically connected with the electrical connection area 122 of the transmitter.

In the embodiment of the invention, the number of metal conductive contacts in the electrical connection area 122 is consistent with the total number of pins 116 (116'). For example, in the stereoscopic three-electrode system, if the number of pins 116 (116') is six, the number of metal conductive contacts in the electrical connection area 122 is also six.

Note that the metal conductive contacts need to be insulated from each other, as well as the connecting circuit between pin 116 (116') and the metal conductive contacts. Therefore, the pins 116 (116') of plane A and plane B need staggered distribution, as shown in solid and dashed lines in Fig.15, so that different pins 116 (116') can connect different conductive areas of the elastic conductor 114, thus realizing the insulation of the connected circuit.

In the embodiment of the invention, the elastic conductor 114 can be one of the materials of conductive adhesive strip, conductive foam or conductive bubble, because these materials such as rubber strip, foam, bubble and so on are electrically insulating, which can ensure good insulation effect between different circuits, and set up a conductive area on the surface of rubber strip, foam and bubble. Alternatively, the conductive area passes through the insulating material in the longitudinal direction of the interval, and the circuit conduction can be realized on all surfaces of the elastic conductor 114.

To sum up, the invention discloses an analyte detection device, the pin is arranged towards the sensor base, the pin is connected with the elastic conductor through the external circuit, and then electrically connected with the electrical connection area of the transmitter. After the orientation of the pin of the signal output end is changed, the side of the substrate without electrode faces the auxiliary needle, and the substrate will bend to the side close to the auxiliary needle. Due to the obstruction of the auxiliary needle, the sensor substrate will no longer bend. When installing the analyte detection device, the auxiliary needle can smoothly pierce the sensor into the user's skin, so as to improve the detection reliability of the sensor.

Although some specific embodiments of the invention have been detailed through examples, technicians in the field should understand that the above examples are for illustrative purposes only and are not intended to limit the scope of the invention. Persons skilled in the field should understand that the above embodiments may be modified without departing from the scope of the invention. The scope of the invention is limited by the attached claims.

## Claims

1. An analyte detection device, comprising:
a transmitter (12) provided with an electrical connection area (122);
a bottom case (10) provided with a sensor base (111);
a sensor (113) comprising a signal output end (113a) and a detection end (113b), wherein the signal output end (113a) is provided with pins (116), the detection end (113b) is provided with electrodes, the pins (116) are connected with the electrodes through wires, the pins (116), the wires and the electrodes are arranged on an insulated substrate (113c) and located on a same side of the substrate (113c);
an elastic conductor (114) comprising at least one conductive area (114a) and an insulating area (114b) distributed at intervals, wherein the conductive area (114a) is electrically connected with the electrical connection area (122),
**characterized in that**
the pins (116) face towards the sensor base (111), the side of the detection end (113b) without electrode faces an auxiliary needle (140), and the pins (116) and the elastic conductor (114) are electrically connected through an external circuit (115).

2. The analyte detection device of claim 1, wherein the elastic conductor (114) is a cuboid structure.

3. The analyte detection device of claim 2, wherein a first surface of the elastic conductor (114) is in contact with the signal output end (113a), a second surface of the elastic conductor (114) is in contact with the external circuit (115), and a third surface of the elastic conductor (114) is in contact with the electrical connection area (122).

4. The analyte detection device of claim 2, wherein a first surface of the elastic conductor (114) is in contact with the signal output end (113a) and the external circuit (115), and a third surface of the elastic conductor (114) is in contact with the electrical connection area (122).

5. The analyte detection device of claim 3, wherein the external circuit (115) is a stereoscopic circuit laid on the sensor base (111).

6. The analyte detection device of claim 4, wherein the external circuit (115) is one of conductive gel, conductive adhesive, conductive coating, conductive tape or conductive glue applied to the sensor base (111).

7. The analyte detection device of any one of claims 3 to 6, wherein the first surface of the elastic conductor (114) is opposite to the third surface.

8. The analyte detection device of claim 1, wherein the conductive area (114a) and the insulating area (114b) pass through the elastic conductor (114) in a longitudinal direction.

9. The analyte detection device of claim 1, wherein the conductive area (114a) and the insulating area (114b) surround a surface of the elastic conductor (114).

10. The analyte detection device of claim 1, wherein the at least one conductive area (114a) comprises elastic conductors distributed at intervals, and the insulating area is the air area between the conductive areas.

11. The analyte detection device of any one of claims 8 to 10, wherein the elastic conductor (114) is one of conductive adhesive strips, conductive foam or conductive bubble.

12. The analyte detection device of claim 1, wherein the signal output end (113a) is bent relative to the detection end (113b), and the signal output end (113a) is laid flat on the sensor base (111).

13. The analyte detection device of claim 1, wherein the electrical connection area (122) comprises at least one metal conductive contact.

14. The analyte detection device of claim 13, wherein a number of the at least one metal conductive contact is consistent with a number of the pins (116).

## Patentansprüche

1. Analytdetektionsvorrichtung, umfassend:
einen Sender (12), der mit einem elektrischen Verbindungsbereich (122) versehen ist;
ein Bodengehäuse (10), das mit einer Sensorbasis (111) versehen ist;
einen Sensor (113), umfassend ein Signalausgangsende (113a) und ein Detektionsende (113b), wobei das Signalausgangsende (113a) mit Pins (116) versehen ist, das Detektionsende (113b) mit Elektroden versehen ist, die Pins (116) über Drähte mit den Elektroden verbunden sind und die Pins (116), die Drähte und die Elektroden auf einem isolierten Substrat (113c) angeordnet sind und sich auf derselben Seite des Substrats (113c) befinden;
einen elastischen Leiter (114), umfassend mindestens einen leitfähigen Bereich (114a) und einen isolierenden Bereich (114b), die in Abständen verteilt sind, wobei der leitfähige Bereich (114a) elektrisch mit dem elektrischen Verbindungsbereich (122) verbunden ist,
**dadurch gekennzeichnet, dass**
die Pins (116) zur Sensorbasis (111) hin weisen, die Seite des Detektionsendes (113b) ohne Elektrode zu einer Hilfsnadel (140) weist und die Pins (116) und der elastische Leiter (114) über eine externe Schaltung (115) elektrisch verbunden sind.

2. Analytdetektionsvorrichtung nach Anspruch 1, wobei der elastische Leiter (114) eine quaderförmige Struktur ist.

3. Analytdetektionsvorrichtung nach Anspruch 2, wobei eine erste Fläche des elastischen Leiters (114) mit dem Signalausgangsende (113a) in Kontakt steht, eine zweite Fläche des elastischen Leiters (114) mit der externen Schaltung (115) in Kontakt steht und eine dritte Fläche des elastischen Leiters (114) mit dem elektrischen Verbindungsbereich (122) in Kontakt steht.

4. Analytdetektionsvorrichtung nach Anspruch 2, wobei eine erste Fläche des elastischen Leiters (114) mit dem Signalausgangsende (113a) und der externen Schaltung (115) in Kontakt steht und eine dritte Fläche des elastischen Leiters (114) mit dem elektrischen Verbindungsbereich (122) in Kontakt steht.

5. Analytdetektionsvorrichtung nach Anspruch 3, wobei die externe Schaltung (115) eine auf der Sensorbasis (111) verlegte dreidimensionale Schaltung ist.

6. Analytdetektionsvorrichtung nach Anspruch 4, wobei die externe Schaltung (115) eines von leitfähigem Gel, leitfähigem Klebstoff, leitfähiger Beschichtung, leitfähigem Band oder leitfähigem Leim ist, das auf die Sensorbasis (111) aufgebracht ist.

7. Analytdetektionsvorrichtung nach einem der Ansprüche 3 bis 6, wobei die erste Fläche des elastischen Leiters (114) der dritten Fläche gegenüberliegt.

8. Analytdetektionsvorrichtung nach Anspruch 1, wobei der leitfähige Bereich (114a) und der isolierende Bereich (114b) den elastischen Leiter (114) in Längsrichtung durchsetzen.

9. Analytdetektionsvorrichtung nach Anspruch 1, wobei der leitfähige Bereich (114a) und der isolierende Bereich (114b) eine Oberfläche des elastischen Leiters (114) umgeben.

10. Analytdetektionsvorrichtung nach Anspruch 1, wobei der mindestens eine leitfähige Bereich (114a) elastische Leiter umfasst, die in Abständen verteilt sind, und der isolierende Bereich der Luftbereich zwischen den leitfähigen Bereichen ist.

11. Analytdetektionsvorrichtung nach einem der Ansprüche 8 bis 10, wobei der elastische Leiter (114) einer von leitfähigen Klebestreifen, leitfähigem Schaum oder leitfähigen Blasen ist.

12. Analytdetektionsvorrichtung nach Anspruch 1, wobei das Signalausgangsende (113a) relativ zum Detektionsende (113b) abgewinkelt ist und das Signalausgangsende (113a) flach auf der Sensorbasis (111) aufliegt.

13. Analytdetektionsvorrichtung nach Anspruch 1, wobei der elektrische Verbindungsbereich (122) mindestens einen metallischen leitfähigen Kontakt umfasst.

14. Analytdetektionsvorrichtung nach Anspruch 13, wobei eine Anzahl des mindestens einen metallischen leitfähigen Kontakts mit einer Anzahl der Pins (116) übereinstimmt.

## Revendications

1. Dispositif de détection d'analyte, comprenant :
un émetteur (12) pourvu d'une zone de connexion électrique (122) ;
un boîtier inférieur (10) pourvu d'une base de capteur (111) ;
un capteur (113) comprenant une extrémité de sortie de signal (113a) et une extrémité de détection (113b), l'extrémité de sortie de signal (113a) étant pourvue de broches (116), l'extrémité de détection (113b) étant pourvue d'électrodes, les broches (116) étant reliées aux électrodes par des fils, et les broches (116), les fils et les électrodes étant agencés sur un substrat isolé (113c) et situés sur un même côté du substrat (113c) ;
un conducteur élastique (114) comprenant au moins une zone conductrice (114a) et une zone isolante (114b) réparties à intervalles, la zone conductrice (114a) étant reliée électriquement à la zone de connexion électrique (122),
**caractérisé en ce que**
les broches (116) sont tournées vers la base de capteur (111), le côté de l'extrémité de détection (113b) dépourvu d'électrode est tourné vers une aiguille auxiliaire (140), et les broches (116) et le conducteur élastique (114) sont reliés électriquement par un circuit externe (115).

2. Dispositif de détection d'analyte selon la revendication 1, dans lequel le conducteur élastique (114) est une structure parallélépipédique.

3. Dispositif de détection d'analyte selon la revendication 2, dans lequel une première surface du conducteur élastique (114) est en contact avec l'extrémité de sortie de signal (113a), une deuxième surface du conducteur élastique (114) est en contact avec le circuit externe (115), et une troisième surface du conducteur élastique (114) est en contact avec la zone de connexion électrique (122).

4. Dispositif de détection d'analyte selon la revendication 2, dans lequel une première surface du conducteur élastique (114) est en contact avec l'extrémité de sortie de signal (113a) et le circuit externe (115), et une troisième surface du conducteur élastique (114) est en contact avec la zone de connexion électrique (122).

5. Dispositif de détection d'analyte selon la revendication 3, dans lequel le circuit externe (115) est un circuit tridimensionnel disposé sur la base de capteur (111).

6. Dispositif de détection d'analyte selon la revendication 4, dans lequel le circuit externe (115) est l'un parmi un gel conducteur, un adhésif conducteur, un revêtement conducteur, un ruban conducteur ou une colle conductrice appliqué sur la base de capteur (111).

7. Dispositif de détection d'analyte selon l'une quelconque des revendications 3 à 6, dans lequel la première surface du conducteur élastique (114) est opposée à la troisième surface.

8. Dispositif de détection d'analyte selon la revendication 1, dans lequel la zone conductrice (114a) et la zone isolante (114b) traversent le conducteur élastique (114) dans une direction longitudinale.

9. Dispositif de détection d'analyte selon la revendication 1, dans lequel la zone conductrice (114a) et la zone isolante (114b) entourent une surface du conducteur élastique (114).

10. Dispositif de détection d'analyte selon la revendication 1, dans lequel l'au moins une zone conductrice (114a) comprend des conducteurs élastiques répartis à intervalles, et la zone isolante est la zone d'air entre les zones conductrices.

11. Dispositif de détection d'analyte selon l'une quelconque des revendications 8 à 10, dans lequel le conducteur élastique (114) est l'un parmi des bandes adhésives conductrices, une mousse conductrice ou des bulles conductrices.

12. Dispositif de détection d'analyte selon la revendication 1, dans lequel l'extrémité de sortie de signal (113a) est coudée par rapport à l'extrémité de détection (113b), et l'extrémité de sortie de signal (113a) est posée à plat sur la base de capteur (111).

13. Dispositif de détection d'analyte selon la revendication 1, dans lequel la zone de connexion électrique (122) comprend au moins un contact conducteur métallique.

14. Dispositif de détection d'analyte selon la revendication 13, dans lequel un nombre de l'au moins un contact conducteur métallique est identique à un nombre des broches (116).
